# EUROPEAN PATENT SPECIFICATION

(11) **EP 3 735 202 B1**
(45) Date of publication and mention of the grant of the patent: **01.05.2024**
(21) Application number: 19736123.1
(22) Date of filing: 02.01.2019
(51) Int. Cl.: A61F 2/24

(54) **STENT FEATURES TO AID WITH APPOSITION AND ALIGNMENT TO NATIVE ANATOMY MITIGATION OF PARAVALVULAR LEAK**
STENTFUNKTIONEN ZUR UNTERSTÜTZUNG BEI DER APPOSITION UND AUSRICHTUNG AN NATIVE ANATOMIEMITIGATION VON PARAVALVULÄREM LECK
ÉLÉMENTS DE STENT D'AIDE À L'APPOSITION ET À L'ALIGNEMENT SUR UNE LIMITATION ANATOMIQUE NATIVE D'UNE FUITE PARAVALVULAIRE

(30) Priority: 02.01.2018 US 201862612836 P; 31.12.2018 US 201816237004
(43) Date of publication of application: 11.11.2020
(73) Proprietor: 4C Medical Technologies, Inc., Maple Grove MN 55311 (US)
(72) Inventor: KRUSE, Steven D., Brooklyn Park, Minnesota 55428 (US); CHAMBERS, Jeffrey W., Brooklyn Park, Minnesota 55428 (US); DIEDERING, Jason S., Brooklyn Park, Minnesota 55428 (US); KUMAR, Saravana B., Brooklyn Park, Minnesota 55428 (US)
(74) Representative: Westphal, Mussgnug & Partner, Patentanwälte mbB
(86) International application number: PCT/US2019/012016
(87) International publication number: WO 2019/136040

(56) References cited:
- WO-A2-2009/042196
- US-A1- 2013 060 329
- US-A1- 2016 213 468
- US-A1- 2017 079 790
- US-B2- 8 083 793
- MILLER, R: "Start-Up Spotlight: 4C Addresses Mitral Regurgitation With Unique 'Dome' Device", MedTech Insight, 29 June 2017 (2017-06-29), XP055619763, Retrieved from the Internet: URL:https://medtech.pharmaintelligence.inf orma.com/MT105076/StartUp-Spotlight-4C-Add resses-Mitral-Regurgitation-With-Unique-Do me-Device

## Description

### FIELD OF THE INVENTION

The invention relates to devices for creating optimal apposition and alignment of a support structure or stent of a prosthetic heart valve to treat cardiac mitral or tricuspid valve regurgitation, mitigating paravalvular leak and optimizing functional efficiency of the prosthetic heart valve.

### DESCRIPTION OF THE RELATED ART

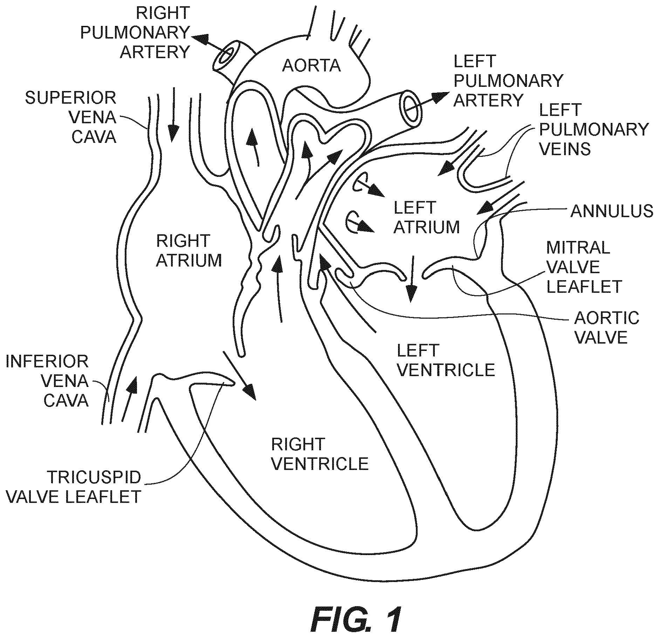

The human heart comprises four chambers and four heart valves that assist in the forward (antegrade) flow of blood through the heart. The chambers include the left atrium, left ventricle, right atrium and left ventricle. The four heart valves include the mitral valve, the tricuspid valve, the aortic valve and the pulmonary valve. Figure 1 illustrates the basic structural features and related blood flow, indicated by arrows, within the human heart.

The mitral valve is located between the left atrium and left ventricle and helps control the flow of blood from the left atrium to the left ventricle by acting as a one-way valve to prevent backflow into the left atrium. Similarly, the tricuspid valve is located between the right atrium and the right ventricle, while the aortic valve and the pulmonary valve are semilunar valves located in arteries flowing blood away from the heart. The valves are all one-way valves, with leaflets that open to allow forward (antegrade) blood flow. The normally functioning valve leaflets close under the pressure exerted by reverse blood to prevent backflow (retrograde) of the blood into the chamber it just flowed out of.

Native heart valves may be, or become, dysfunctional for a variety of reasons and/or conditions including but not limited to disease, trauma, congenital malformations, and aging. These types of conditions may cause the valve structure to either fail to properly open (stenotic failure) and/or fail to close properly (regurgitant).

Mitral valve regurgitation is a specific problem resulting from a dysfunctional mitral valve. Mitral regurgitation results from the mitral valve allowing at least some retrograde blood flow back into the left atrium from the left ventricle. This backflow of blood places a burden on the left ventricle with a volume load that may lead to a series of left ventricular compensatory adaptations and adjustments, including remodeling of the ventricular chamber size and shape, that vary considerably during the prolonged clinical course of mitral regurgitation.

A similar problem may occur when the tricuspid valve weakens or begins to fail. The tricuspid valve separates the right atrium and the right ventricle. Tricuspid regurgitation, also known as tricuspid insufficiency, occurs when the tricuspid valve doesn't close properly, causing blood to flow back up into the right atrium when the right ventricle contracts. Various embodiments of the present invention discussed herein may apply to treatment of mitral valve regurgitation or tricuspid valve regurgitation. Further, embodiments of the present invention may be used to treat and/or prevent mitral and/or tricusid stenosis caused by calcification. The present invention may be used to crack and/or open the stenosis to improve and/or maintain blood flow through the valve.

Native heart valves generally, i.e., mitral valves or tricuspid valves, therefore, may undergo functional repair, including a partial or complete replacement using known methods and devices. Such intervention may take several forms including open heart surgery or open heart implantation of a replacement heart valve. See e.g., U.S. Pat. No. 4,106,129 (Carpentier), for a procedure that is highly invasive, fraught with patient risks, and requiring not only an extended hospitalization but also a highly painful recovery period.

Less invasive methods and devices for replacing a dysfunctional heart valve are also known and involve percutaneous access and catheter-facilitated delivery of the replacement valve. Most of these solutions involve a replacement heart valve attached to a structural support such as a stent, commonly known in the art, or other form of wire network designed to expand upon release from a delivery catheter. See, e.g., U.S. Pat. No. 3,657,744 (Ersek); U.S. Pat. No. 5,411,552 (Andersen). The self-expansion variants of the supporting stent assist in positioning the valve, and holding the expanded device in position, within the subject heart chamber or vessel. This self-expanded form also presents problems when, as is often the case, the device is not properly positioned in the first positioning attempt and, therefore, must be recaptured and positionally adjusted. This recapturing process in the case of a fully, or even partially, expanded device requires re-collapsing the device to a point that allows the operator to retract the collapsed device back into a delivery sheath or catheter, adjust the inbound position for the device and then re-expand to the proper position by redeploying the positionally adjusted device distally out of the delivery sheath or catheter. Collapsing the already expanded device is difficult because the expanded stent or wire network is generally designed to achieve the expanded state which also resists contractive or collapsing forces.

Besides the open heart surgical approach discussed above, gaining access to the valve of interest is achieved percutaneously via one of at least the following known access and delivery routes: femoral access, venous access, trans-apical, trans-aortic, trans-jugular, trans-caroticd, trans-septal, trans-atrial, retrograde from the aorta delivery techniques.

Generally, the art is focused on systems and methods that, using one of the above-described known access routes, allow a partial delivery of the collapsed valve device, wherein one end of the device is released from a delivery sheath or catheter and expanded for an initial positioning followed by full release and expansion when proper positioning is achieved. See, e.g., U.S. Pat. Nos. 8,852,271 (Murray, III); 8,747,459 (Nguyen); 8,814,931 (Wang); 9,402,720 (Richter); 8,986,372 (Murray, III); and 9,277,991 (Salahieh); and U.S. Pat. Pub. Nos. 2015/0272731 (Racchini); and 2016/0235531 (Ciobanu).

However, known delivery systems, devices and methods still suffer from significant flaws in delivery methodology including, *inter alia,* positioning and recapture capability and efficiency.

In addition, known "replacement" prosthetic heart valves are intended for full replacement of the native heart valve. Therefore, these replacement heart valves physically engage tissue within the annular throat, i.e., below the annular plane and upper annular surface, and/or valve leaflets, thereby eliminating all remaining functionality of the native valve and making the patient completely reliant on the replacement valve. Generally speaking, it is a preferred solution that maintains and/or retains the native function of a heart valve, thus supplementation of the valve is preferred rather than full replacement. Obviously, there will be cases when native valve has either lost virtually complete functionality before the interventional implantation procedure, or the native valve continues to lose functionality after the implantation procedure. The preferred solution is delivery and implantation of a valve device that will function both as an adjunctive and/or supplementary functional valve as well as be fully capable of replacing the native function of a valve that has lost, or will lose, most or all of its functionality. However, the inventive solutions described *infra* will apply generally to all types and forms of heart valve devices, unless otherwise specified.

Further, known solutions for, e.g., the mitral valve replacement systems, devices and methods require 2-chamber solutions, i.e., there is involvement and engagement of the implanted replacement valve device in the left atrium and the left ventricle. Generally, these solutions include a radially expanding stent in the left atrium, with anchoring or tethering (disposed downward through the native annulus or annular throat) connected from the stent device down through the annular throat, with the sub-annular surface within the left ventricle, the left ventricular chordae tendineae and even into the left ventricle wall surface(s). *See, e.g.,* the MitraClip^{®} marketed by the Abbott Group and currently the only US approved repair device. With the MitraClip^{®} a catheter containing the MitraClip^{®} is inserted into the femoral vein. The device enters the heart through the inferior vena cava to the right atrium and delivered trans-septally. The MitraClip^{®} passes through the annulus into the left ventricle and sits below the leaflets, clipping the leaflets to decrease regurgitation.

Such 2-chamber and native annulus solutions are unnecessary bulky and therefore more difficult to deliver and to position/recapture/reposition from a strictly structural perspective. Further, the 2-chamber solutions present difficulties in terms of making the ventricular anchoring and/or tethering connections required to hold position. Moreover, these solutions interfere with the native valve functionality as described above because the device portions that are disposed within the left ventricle must be routed through the native annulus and/or annular throat and native mitral valve, thereby disrupting any remaining coaptation capability of the native leaflets. In addition, the 2-chamber solutions generally require an invasive anchoring of some of the native tissue, resulting in unnecessary trauma and potential complication.

It will be further recognized that the 2-chamber mitral valve solutions require sub-annular and/or ventricular engagement with anchors, tethers and the like precisely because the atrial portion of the device fails to adequately anchor itself to the atrial chamber and/or upper portion of the annulus. Again, some of the embodiments, or portions thereof, described herein are readily applicable to single or 2-chamber solutions, unless otherwise indicated.

Finally, known prosthetic cardiac valves consist of two or three leaflets that are arranged to act as a one-way valve, permitting fluid flow therethrough in the antegrade direction while preventing retrograde flow. The native mitral valve is located retrosternally at the fourth costal cartilage, consisting of an anterior and posterior leaflet, chordae tendineae, papillary muscles, ventricular wall and annulus connected to the atria. Each native leaflet is supported by chordae tendineae that are attached to papillary muscles which become taut with each ventricular contraction preserving valvular competence. Both the anterior and posterior leaflets of the native valve are attached via primary, secondary and tertiary chordae to both the antero-lateral and posterio-medial papillary muscles. A disruption in either papillary muscle in the setting of myocardial injury, can result in dysfunction of either the anterior or posterior leaflet of the mitral valve. Other mechanisms may result in failure of one, or both of the native mitral leaflets. In the case of a single mitral valve leaflet failure, the regurgitation may take the form of a non-central, eccentric jet of blood back into the left atrium. Other leaflet failures may comprise a more centralized regurgitation jet. Known prosthetic valve replacements generally comprise leaflets which are arranged to mimic the native valve structure, which may over time become susceptible to similar regurgitation outcomes.

Publication US 2017/079790 A1 discloses a prosthetic mitral valve comprising an inner frame and an outer frame that are connected to each other at various coupling points.

As discussed above, known delivery methods and devices comprise expandable prosthetic valves that are collapsed during delivery via a delivery catheter. The problems with such collapsing and expanding structures include placing strain on the regions of the structure, e.g., stent, that must bend to accommodate the collapsing and expanding states. Further, the collapsed geometry in known devices may not be controlled or predictable, adding to the strain on the collapsing and expanding structure elements.

In addition, known prosthetic mitral valves may be improved upon in terms of sealing and protecting against paravalvular leakage from the left ventricle to the left atrium as well as the attachment and alignment of the leaflets to the support structure.

Various embodiments of the present invention address these, *inter alia,* issues.

### BRIEF SUMMARY OF THE INVENTION

The invention is set out in the appended claims. The application pertains to an expandable and collapsible stent comprising prosthetic leaflets attached to an inner valve support section that extends radially upward into an outer stent section, the stent adapted for use in treating a dysfunctional native heart valve, including the mitral valve, the tricuspid valve and the aortic valve. A transition stent section is disposed between the inner valve support section and the outer stent section. Each of the outer stent section, the inner valve support section and the transition stent section may comprise struts or the equivalent that form and define cells having a pattern, wherein each section may comprise a different cell pattern. Transition stent section preferably comprises struts that are of equal curvature, with adjacent struts equally spaced from each other to allow nested collapsing of the transition stent section struts. A boss section extending downstream away from the inner valve support section may be provided attached to, or integrated with, the outer stent section or the transition stent section to aid in alignment and retention of the expanded stent and may comprise a shape that is complementary to a heart chamber annulus.

### BRIEF DESCRIPTION OF THE DRAWINGS

FIG. 1 is a cross-sectional view of the human heart and related blood vessels and valves.
FIG. 2 is a perspective view of one embodiment of the present invention.
FIG. 3A is a bottom view of one transition section embodiment of the present invention.
FIG. 3B is a bottom view of one transition section embodiment of the present invention.
FIG. 3C is a bottom view of one transition section embodiment of the present invention.
FIG. 4A is a bottom view of one transition section embodiment under compression of the present invention.
FIG. 4B is a bottom view of one transition section embodiment under compression of the present invention.
FIG. 5 is a side cutaway view of a mitral valve annulus.
FIG. 6 is a bottom cutaway view of one embodiment of the present invention.
FIG. 7 is a side view of one embodiment of the present invention.
FIG. 8A is a perspective view of one embodiment of the present invention.
FIG. 8B is a perspective view of one embodiment of the present invention.
FIG. 9 is a side view of one embodiment of the present invention.
FIG. 10 is a bottom view of one embodiment of the present invention.
FIG. 11 is a side cutaway view of one embodiment of the present invention.
FIG. 12 is a side cutaway view of one embodiment of the present invention.

### DETAILED DESCRIPTION OF THE INVENTION

While the invention is amenable to various modifications and alternative forms, specifics thereof are shown by way of example in the drawings and described in detail herein. It should be understood, however, that the intention is not to limit the invention to the particular embodiments described. On the contrary, the intention is to cover all modifications, equivalents, and alternatives falling within the scope of the invention.

The following description refers generally throughout to the anatomical structures and related blood flow illustrated in Fig. 1.

Generally, various embodiments of the present invention are directed to devices for creating optimal apposition of a support structure or stent of a prosthetic heart valve to treat cardiac mitral or tricuspid valve regurgitation, mitigating paravalvular leak and optimizing functional efficiency of the prosthetic heart valve.

The support structure ( an expandable stent) has multiple functions to aid with the treatment of cardiac valve regurgitation (mitral or tricuspid). These functions include its function as a scaffold for the functioning prosthetic valve and associated leaflets, apposition to the atrial anatomy, optimized radial force for compliance with atrial distension, ability to load and deploy from a minimally invasive delivery system, and geometry to support with mitigating against paravalvular leak (PVL). The design features of the stent are adapted to meet one or more of the functions identified above. Specific design features and attributes for the stents are discussed in detail below.

### Stent Design Concepts

The stent design concepts are intended to support minimally invasive procedures for the treatment of valvular regurgitation - mitral, tricuspid and/or otherwise. The stents may be self-expandable (e.g. nitinol or similar materials) or balloon expandable (e.g. cobalt chromium or similar materials) as is known in the art. The stent may be made of cells that may be open celled diamond like structures or continuous structures that have a working cell element. The stents may also be constructed using tubing, wires, braids or similar structures. Specific design features that aid with the functioning of the stent are described in detail below.

### Stent "Iris" transition cells

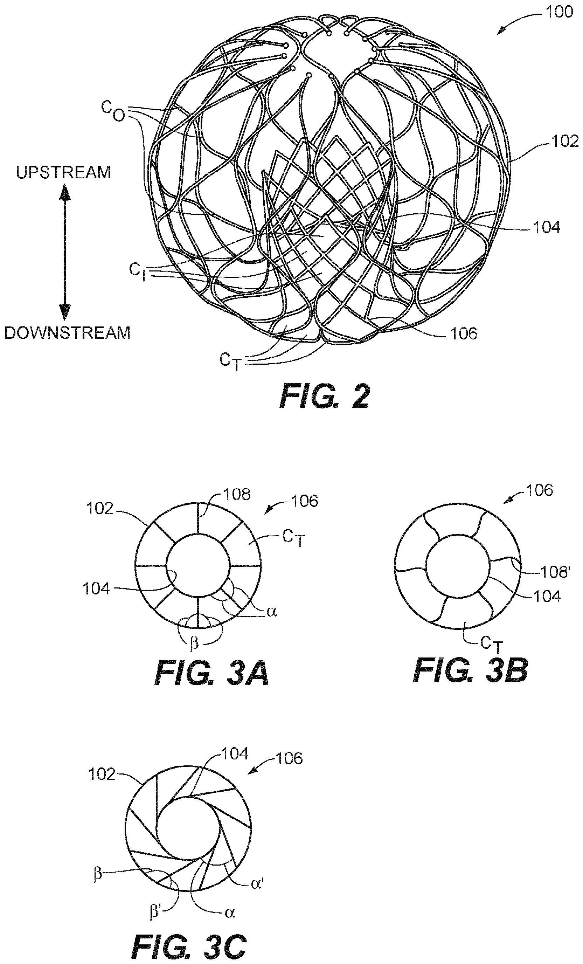

With reference now to Figures 2-3B, one embodiment of the stent 100 of the present invention comprises an outer section 102 - that may generally be circular though need not be a perfectly round circular structure when fully and/or partially expanded - and an inner valve support section 104 - which may be cylindrical but need not be a constant diameter cylinder and is adapted to support and retain prosthetic valve leaflets (not shown in Fig. 2) within the inner valve support section 104, most preferably at a point that located above the native annulus, e.g., the mitral valve annulus, though other attachment points for the prosthetic leaflets are within the scope of the present invention. Further, as discussed above, the stent 100 may be configured to supplement and/or replace the function of the tricuspid valve. A preferred construction comprises the prosthetic leaflets disposed above the native leaflets, wherein the prosthetic leaflets are attached and spaced sufficiently away from (above) the native leaflets so as to not physically interfere or interact with the native leaflets. However, certain embodiments contemplate some interaction with the native leaflets.

Individual cells Co forming the outer section 102 of stent 100 are visible in Figure 2 as open cell regions defined by the material used to form the expandable stent 100.

Individual cells Cr forming the inner valve support section 104 are also illustrated as open cells regions formed within an inner region R defined by outer section 102, wherein the inner valve support section extends radially upward into the inner region R. As shown, individual cells Cr are of a different size, and may comprise a different shape, than that of individual cells Co.

The region of stent 100 that facilitates the radially inward transition of the stent 100 from the outer section 102 to the inner section 104 of the stent 100 is the transition cell region 106. Transition cell region 106 may comprise cells C_{T} that may comprise a different size and/or shape that either the outer section cells Co and/or the inner section cells C_{I}. The outer and/or inner regions 102, 104, and/or transition cell region 106 of the stent 100 may be constructed from one continuous structure or may combine two or more structures to achieve intended design goals. Transition cell region 106 comprises generally a radially upward turn to allow the inner valve support section 104 to reside within the inner region 102 as shown in Fig. 2. In some embodiments, the lower portion of inner valve support section 104, that is the portion of the inner valve support section 104 that is in connection with the cells C_{T} of transition cell region 106 may also comprise a curving shape to facilitate and/or complete the radially upward turn into the inner region 102.

The geometry and/or shape of the transition cells C_{T} may be substantially straight segments when expanded as in Figure 3A below or may, as shown in Figure 3B, incorporate an offset or a twist in the stent cell pattern when expanded to allow for a controlled compression of the stent. Exemplary cross-sectional geometry of the transition cell region 106 viewed from the bottom of stent 100 is represented schematically in Figures 3A and 3B.

This transition cell region 106 of the stent 100 may be a strut, completed cell section or a partial cell section. The transition cell region 106 may have any number of struts (minimum of 3) or cell sections as generally required to meet design needs. Transition cells C_{T} or struts may be evenly spaced and formed by substantially straight and equally spaced apart struts 108 as shown in Fig. 3A, that extend away from the inner valve support section 104 with equal angles α on both sides of the strut 108 and equal angles β on both sides of strut 108 with respect to its intersection or integration with outer support section 102.

In a preferred embodiment, the struts 108 of transition section 106 may be straight as in Figure 3A, but with non-equal angles relative to the inner valve support section 104 and outer support section 102 as shown in Figure 3C. There, the straight struts 108 are slanted so that a smaller angle α and a larger angle α' are provided relative to the inner valve support section 104. Similarly, a smaller angle β' and a larger angle β are provided relative to the outer support section 102. This allows a compressed nesting of the slanted struts 108 of transition section 106.

In another preferred embodiment, the transition cell region 106 may comprise transition cell struts 108' that comprise transition cells CT that are formed by struts 108' having an offset, i.e., not straight, are twisted and/or curvilinear. The degree of offset and/or twist and/or curvature of the struts 108', and therefore the size and/or shape of the resultant expanded cells CT may be varied dependent on the number of cells/struts in the transition cell region 106, packing density when the stent is collapsed, and stress/strain distribution limitations of the transition cell region 106.

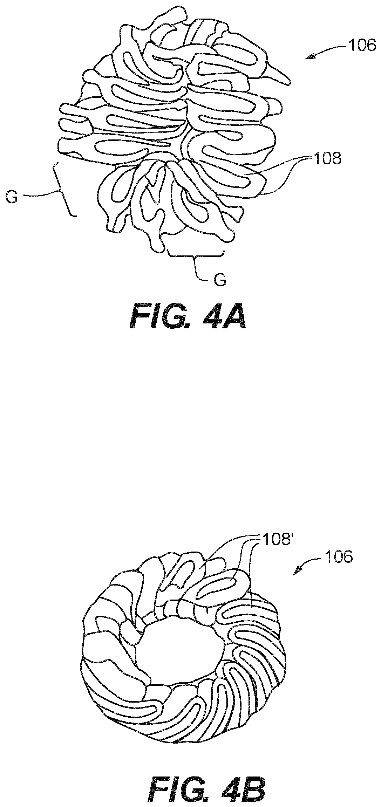

The structure of Figures 3B and 3C are preferred over the straight transition cell region 106 structure of Fig. 3A for several reasons. Figure 4A shows a transition cell region 106 in a collapsed form using the substantially straight struts 108 of Fig. 3A and with, undesirable, gaps G between selected struts 108. Though this resultant gapping collapsed transitional cell region 106 is workable, it is not optimal.

Thus, the transition section 106 of Figure 4B, using e.g., the offset and/or twisted and/or curved plurality of struts 108' of Fig. 3B or the slanted straight struts 108 of Fig. 3C, allows for a controlled and predictable collapsed form of the stent, without gapping between the struts 108'. This, in turn, minimizes the amount of stress/strain concentration at the lower region of the stent 100 during collapsing as is required for delivery of the expandable stent 100 to the heart region of interest. Additionally, the collapse of the cells is also symmetrical and uniform, which could aid with mitigating against damage to the valve tissue or fabric when it is attached to the stent cells. Reduction in overall stress/strain of the transition strut section may benefit the durability of the stent and the valve tissue.

A feature of certain embodiments of the transition cell region 106 as shown in Figures 3B and 3C and 4B, i.e., with offset, twisted and/or curved struts 108' or slanted straight struts 108, is that, as best shown in Figure 3B, the struts 108' each comprise the same offset, twist and/or curvature. This, in turn, enables a close nesting of adjacent struts 108' as the stent 100 is collapsed down for delivery and subsequent expansion.

### Stent geometry - Transition section

The geometry of the native valve annulus is typically D-shaped, saddle shaped or oval depending on the diseased state of the patient. The transition cell region 106 of the stent100 interacts with the valve annulus, e.g., the mitral valve annulus, to ensure that the positioning allows for normal forward flow of blood while preventing regurgitant blood flow from entering the left atrium. The transition cell region 106 of the stent 100 may be located upon expansion either supra-annular, at the annulus or sub-annular while the functioning prosthetic valve stays at or above the native valve annulus, thereby minimizing and, in certain embodiments, eliminating physical interaction between the native valve leaflets and the prosthetic valve leaflets.

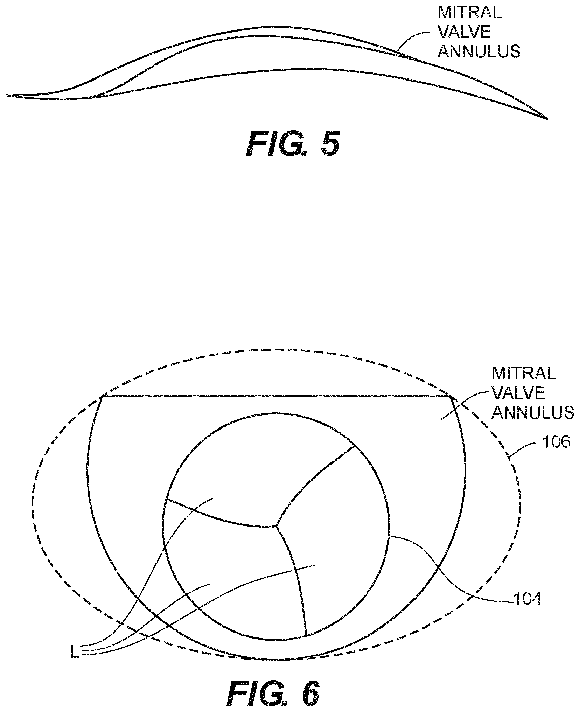

As shown in Fig. 5, below, the exemplary mitral valve annulus may comprise a saddle shape as viewed from the side. Thus, transition cell region 106 may comprise a complementary shape to fit sealingly against the saddle-shaped mitral valve annulus and/or may comprise a degree of compliance that enables the transition cell region 106 to adaptingly seal against the saddle-shaped annulus as the self-expanding stent 100 is allowed to expand within the exemplary left atrium.

Figure 6, viewing the annulus from below the exemplary mitral valve annulus, illustrates the mitral valve annulus having a substantially "D" shaped opening. Thus, the shapes and/or geometry of the transition cell region 106 and the outer stent section 102 are critical in ensuring alignment of the implant to the native valve, and for mitigation of PVL. Figure 6 illustrates the expanded transition cell region 106 in the outer dashed line, extending at least to the boundaries at all locations along the D-shaped mitral valve annulus. In this way, and in some cases in combination with the complementary shaping of transition cell region 106 discussed above in connection with Fig. 5, the transition cell region 106 also assists in the optimization of the positioning and/or alignment of the inner valve support 104, and valve leaflets L attached thereto, over the exemplary mitral valve annulus to help optimize blood flow (and blockage of regurgitant) therethrough. In addition, the transition cell region 106, properly positioned as discussed herein, works to minimize and/or eliminate any paravalvular leakage (PVL) from the left ventricle back into the left atrium.

Any one or more of the design concepts discussed below may be reasonably combined to achieve intended design function.

### Circular/Oval profile

The transition cell region 106 of the stent 100 may be circular or ovalized to provide adequate oversizing of the implant to the native valve annulus. The aspect ratio of the oval may vary to accommodate the dynamics of the native valve annulus. *See, e.g.,* Figure 6.

### D-shaped profile

The transition cell region 106 of the stent 100 may be D-shaped to match the shape of or oversized accordingly to the native annulus. The cells of the stent are profiled where either the stent struts or cells may be shape set to retain the D-shape. *See* Fig. 6.

### Boss/extended profile

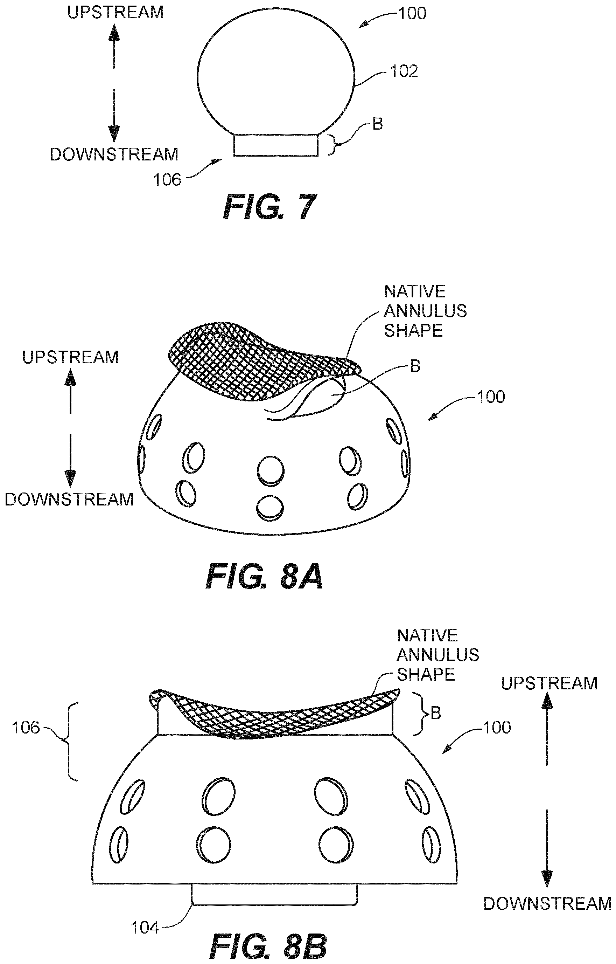

As shown in Figure 7, a boss section B may be provided integrated with, or attached to, the transition cell region 106, wherein the boss section B extends away from the outer stent section 102 in a downstream direction such that the stent seats itself at the level of annulus or subannular, with the boss section B extending downward slightly into the annular throat, and radially expanding in some cases against the inner portion of the annular throat. Alternatively, boss section B may be attached to, or integrated with, outer section 102. The boss section B allows the stent 100 to maintain position/alignment with respect to the native valve leaflets while the transition to the outer stent section 102 may be used to mitigate against PVL. The dimensions of the boss section B (length) may be equal to or less than the maximum diameter (or width) of the outer section 102 of the stent 100 and may be oversized and/or varied to provide radial forces against the portion of the annular throat engaged by boss section B when stent 100 and boss section B are expanded.

The boss section B may, similar to the shaping considerations relating to the transition cell region 106 discussed above in connection with Figures 5 and 6, comprise the following design considerations, whether boss section B is integrated with or attached to transition section 106 or outer section 102 of stent 100:

### Circular/oval profile

The aspect ratio and height of the boss section B may be varied within reasonable limits, and may in some cases extend downward into the annular throat, i.e., sub-annularly positioned. In some embodiments, the boss section B may be slightly oversized and/or varied relative to the native mitral valve orifice or throat, with radial forces pressing on the annular through when the boss section B is expanded to provide additional alignment force and presence as well as PV mitigation.

### D-shaped profile

The boss section B may comprise a D-shape to match the D-shaped exemplary mitral valve annulus, in some embodiments, the length of the D-shaped boss section B may be varied to match, or oversize, the annulus dimensions.

### 3D saddle shape

The profile of the extended boss section B whether attached to or integrated with transition section 106 or outer section 102 may be varied in three dimensions to match with the saddle-shaped profile of the native annulus. The height/depth of the boss section may be contoured to vary along the length and/or width of the profile to form a complementary profile and fit for the native annulus. For example, for the D-shaped boss section B, the ends of the boss section B may have more depth/height as compared to closer to the center to form the desired complementary shaping. This may allow the profile of the stent 100 to sit well into the commissures for alignment as well as mitigation of PVL. In some embodiments, only the ends with greater depth/height may extend into the native annular throat, i.e., subannularly. A representation of such a profile is provided in Figures 8A and 8B which are inverted relative to the other stent figures to provide better view of the boss section B. The mesh-like structure is depicting the native upper annular surface shape, with complementary contouring of the boss section B to provide a relatively fit to assist in aligning the stent 100 as well as prevention of PVL.

### Flared profile

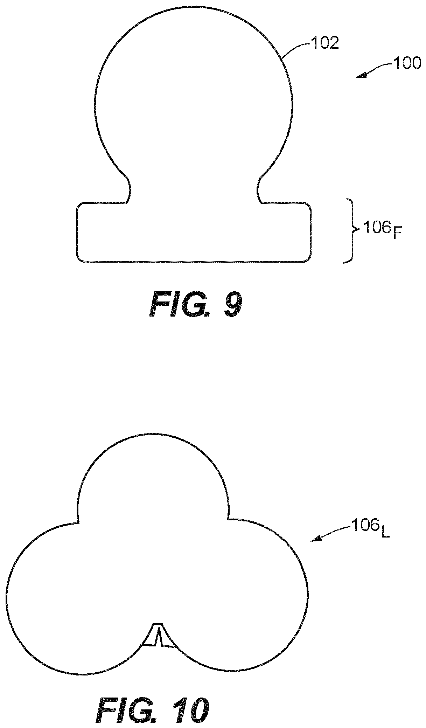

The boss section B, whether attached to or integrated with transition cell section 106 or outer section 102 of the stent 100 may be flared to seat itself at the level of the native exemplary mitral annulus and/or subannular, i.e., at least partially disposed below the upper annular surface and therefore within the annular throat, for positioning and alignment of the stent 100, and in particular the prosthetic leaflets L, with respect to the native valve leaflets. The diameter (or length) of the flared transition cell region 106_{F} may be equal to or greater than the maximum diameter (or width) of the outer section 102 of the stent. A representation of such a profile is provided in Figure 9. The height/depth of the flared section 106_{F} may be varied to accommodate variability in expected native valve geometries.

### Lobular Profile

The boss section B, whether attached to or integrated with transition cell region 106 or outer section 102 of the stent 100 may be shaped like lobes 106_{L}. A representation of such three-lobe structure is provided in Fig. 10, as viewed from the bottom of the transition cell region 106_{L}. The dimensions of the lobe may be varied to match or accommodate various geometries of the native human valve. A boss/extension B and/or a flare 106_{F} may be added to the lobe(s) of the lobular transition cell region 106_{L} and/or outer section 102. Each of the lobes of the lobular transition cell region 106_{L} may have similar dimensions or may have different dimensions. The ends of the lobes are connected to maintain its continuity. The geometry of the individual and/or all lobes of the lobular transition cell region 106_{L} may be varied (e.g. flat sections at the top of lobes, extended arms of the lobes, non-spherical lobes) to accommodate design needs.

In addition to aiding implant positioning/alignment and mitigation of PVL, the lobular design is useful as a mechanism to reduce the closing volume flow needed for the closure of the implant leaflets. Typically, there is a minimum amount of regurgitant flow or volume across the native leaflets needed for closure of leaflets (either native or implanted valves). The lobes of the stent geometry - similar to a coronary sinus function for aortic valve replacement leaflets - directs flow behind the implanted tissue valve leaflets and aids with closing the leaflets at lower closing volume and/or pressure.

### Stent geometry - Outer section 102

The outer section 102 of the stent 100 assists with engaging the exemplary left atrium by oversizing and prevents embolization and/or migration of the implant. The compliance of the stent 100 and outer section 102 may be tailored to meet with the compliance of the atrial anatomy, and varied to accommodate expected variations in anatomy. The geometries of the outer stent section 102 may be designed to accommodate variabilities of the atrial anatomy.

### Specific designs of the outer section 102 are discussed below.

The outer section 102 of the stent 100 may comprise a circular shape that may have a round shape or may comprise a non-round circular shape, whose diameter may be varied to accommodate expected variations in human atrial or other heart chamber anatomy.

The outer section 102 of the stent 100 may be oval with a combination of aspect ratios for the major and minor diameter to accommodate expected variations in human atrial or other heart chamber anatomy.

The bottom section of the stent 100, i.e., the transition cell region 106 and its various configurations discussed above, and/or the boss section B, may be flat, convex, concave or slanted to accommodate expected variations in human atrial or other heart chamber anatomy.

The top of the outer region 102 of the stent 100 may be flat, convex, concave or slanted to promote better contact and apposition to the atrial or other heart chamber anatomy.

### Stent features for attachment

Various features are incorporated into the stent 100 to assist with (but not limited to) valve attachment, load distribution, fabric attachment, repositioning, reorientation, and recapture of the stent 100. Specific features are discussed below.

### Leaflet Extension Attachment Feature (LEAF)

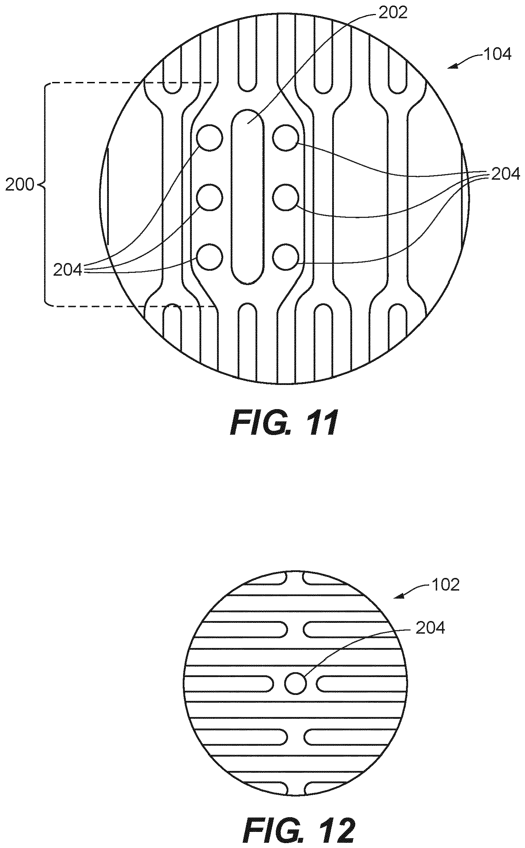

With reference now to Figures 11 and 12, the ends of the leaflets L need to be reliably attached to the stent frame to ensure that the load distribution may occur efficiently between the valve leaflet and the stent. The valve tissue leaflet ends need to be folded over the inner valve support section 104 for attachment. The LEAF 200 is designed to capture these leaflet ends and fold them over the outside of the inner valve support section 104 of the stent 100. The slot 202 at the center of the LEAF is designed to allow the leaflet ends to pass through slot 202 and fold on the outside of the inner valve support section 104 of the stent 100. The holes or apertures or eyelets 204 in the LEAF 200 allow for sutures to attach the valve leaflet L to the stent 100.

### Stent Eyelets/slots

As discussed, one or more eyelets 204 and/or slots 202 may be incorporated into the stent to assist with valve attachment, fabric attachment for PVL mitigation, delivery system attachment for repositioning/reorientation of the implant, and recapture of the implant during or after deployment.

Figure 12 shows a representation of an eyelet feature 204 added to the laser cut profile of the outer section 102 of the stent to aid with fabric attachment. The number and location of the eyelet(s) 204 may vary to accommodate design needs. The size, dimensions, and geometry of the eyelet(s) 204 may also be varied reasonably. These eyelets 204 may also be extended to become slots 202 as needed. These features may be included in any section of the stent 100 - outer section 102, inner valve support section 104 and/or at the transition section 106 - without limitations. The location of the eyelets 204 is generally positioned in between the stent cells C at the junction of struts 108, 108' (also defined as nodes). However, they may be incorporated along the length of the stent struts 108, 108' or added as features in between stent cells as needed.

The description of the invention and its applications as set forth herein is illustrative and is not intended to limit the scope of the invention.

These and other variations and modifications of the embodiments disclosed herein may be made without departing from the scope of the invention.

## Claims

1. A collapsible and expandable stent (100) adapted to treat blood flow regurgitation in a native heart valve by allowing downstream blood flow and preventing upstream blood flow, the stent comprising:
an outer section (102) comprising a first stent cell pattern;
an inner valve support section (104) extending radially upward into the outer section (102) and comprising the first stent cell pattern or a second stent cell pattern, the inner valve support section (104) supporting prosthetic valve leaflets attached therein;
a transition section (106) between the outer section (102) and the inner valve support section (104) comprising a third stent cell pattern that is different from the first stent cell pattern and the second stent cell pattern, wherein the transition section (106) turns radially inward to give rise to a radially upward extension of the inner valve support section (104) within the outer section (102),
wherein the third stent cell pattern of the transition section (106) further comprises a plurality of curvilinear struts (108') comprising a degree of curvature and/or twist,
wherein a spacing between adjacent curvilinear struts (108') defines the cells of the transition section (106) and the third stent cell pattern, and
wherein adjacent curvilinear struts (108') are configured to nest together when the stent (100) is collapsed.

2. The stent (100) of claim 1,
wherein the degree of curvature and/or twist is adapted to be the same for each of the plurality of curvilinear struts (108'), and
wherein the spacing between adjacent curvilinear struts (108') is constant or equal across the plurality of curvilinear struts (108').

3. The stent (100) of claim 1, wherein the degree of curvature and/or twist is adapted to differ or vary for at least one of the plurality of curvilinear struts (108').

4. The stent (100) of claim 1, wherein the spacing between adjacent curvilinear struts (108') varies for at least one pair of adjacent curvilinear struts (108').

5. The stent (100) of claim 1, wherein the third stent cell pattern of the transition section (106) further comprises a plurality of straight struts (108) and a spacing between adjacent straight struts (108) defining the cells of the transition section (106) and the third stent cell pattern.

6. The stent (100) of claim 5, wherein the plurality of straight struts (108) are slanted with respect to the inner valve support section (104), and wherein adjacent slanted straight struts are adapted to nest together when the stent is collapsed.

7. The stent (100) of claim 1, wherein the transition section (106) comprises at least one expanded shape selected from the group consisting of: circular, oval, elliptical, or D-shaped.

8. The stent (100) of claim 1, wherein the transition section (106) comprises a flared profile comprising a diameter that is equal to or greater than an expanded maximum diameter of the outer section (102) of the stent (100).

9. The stent (100) of claim 1, wherein the transition section (106) comprises a plurality of lobe-shaped elements.

10. The stent (100) of claim 9, wherein the stent is adapted to treat a native heart valve comprising native leaflets and wherein the number of lobe-shaped elements is matched to a number of the native leaflets in the native valve.

11. The stent (100) of claim 9, wherein the lobe-shaped elements of the transition section (106) are adapted to direct blood flowing in a regurgitating upstream direction through the native heart valve across native leaflets in a manner that reduces the closing volume and/or pressure required to close the prosthetic valve leaflets attached within the inner valve support section (104).

12. The stent (100) of claim 1, wherein the outer section (102) has a top section of an expanded shape selected from the group consisting of: flat, convex, concave or slanted.

13. The stent (100) of claim 1, further comprising:
at least two slots (202) defined and formed by adjacent struts forming the second cell structure pattern of the inner valve support section (104), each slot adapted for receiving an inner end of at least one of the prosthetic valve leaflets therethrough and further adapted to align, and maintain alignment, of the prosthetic valve leaflet within the inner valve support section (104).

14. The stent (100) of claim 13, further comprising:
one or more eyelets (204) through each adjacent strut defining and forming each of the at least two slots (202), each eyelet adapted for suturing the inner end of the prosthetic valve leaflet thereto.

15. The stent (100) of claim 13, further comprising:
three eyelets (204) through each adjacent strut that form and define each of the at least two slots (202),
wherein the three eyelets (204) in each adjacent strut define three pairs of eyelets, and
wherein a slot (202) is positioned between the adjacent struts and the three pairs of eyelets defined by the adjacent struts.

16. The stent (100) of claim 1,
wherein the outer section (102) comprises a plurality of struts defining the first stent cell pattern, and
wherein at least one fabric attachment eyelet or aperture is defined through at least one of the plurality of struts of the outer section (102).

17. The stent (100) of claim 1, wherein the stent (100) comprises a prosthetic mitral valve or a prosthetic tricuspid valve.

18. The stent (100) of claim 1, wherein the outer section (102), the transition section (106) and the inner valve support section (104) comprise one continuous structure.

## Patentansprüche

1. Zusammenfaltbarer und ausdehnbarer Stent (100), der angepasst ist, um eine Blutflussregurgitation in einer nativen Herzklappe zu behandeln, indem er einen stromabwärtigen Blutfluss zulässt und einen stromaufwärtigen Blutfluss verhindert, wobei der Stent Folgendes umfasst:
einen äußeren Abschnitt (102), der ein erstes Stentzellenmuster umfasst;
einen inneren Klappenträgerabschnitt (104), der sich radial nach oben in den äußeren Abschnitt (102) erstreckt und das erste Stentzellenmuster oder ein zweites Stentzellenmuster umfasst, wobei der innere Klappenträgerabschnitt (104) darin angebrachte prothetische Klappensegel trägt;
einen Übergangsabschnitt (106) zwischen dem äußeren Abschnitt (102) und dem inneren Klappenträgerabschnitt (104), der ein drittes Stentzellenmuster umfasst, das sich von dem ersten Stentzellenmuster und dem zweiten Stentzellenmuster unterscheidet, wobei der Übergangsabschnitt (106) radial nach innen abbiegt, um eine radial nach oben gerichtete Erstreckung des inneren Klappenträgerabschnitts (104) innerhalb des äußeren Abschnitts (102) zu bewirken,
wobei das dritte Stentzellenmuster des Übergangsabschnitts (106) ferner eine Vielzahl von kurvenförmigen Streben (108') umfasst, die einen Grad einer Krümmung und/oder Wendung umfasst,
wobei ein Abstand zwischen benachbarten kurvenförmigen Streben (108') die Zellen des Übergangsabschnitts (106) und des dritten Stentzellenmusters definiert und
wobei benachbarte kurvenförmige Streben (108') dazu konfiguriert sind, ineinander verschachtelt zu sein, wenn der Stent (100) zusammengefaltet ist.

2. Stent (100) nach Anspruch 1,
wobei der Grad der Krümmung und/oder Wendung so angepasst ist, dass er für jede der Vielzahl von kurvenförmigen Streben (108') gleich ist, und
wobei der Abstand zwischen benachbarten kurvenförmigen Streben (108') über die Vielzahl von kurvenförmigen Streben (108') konstant oder gleich ist.

3. Stent (100) nach Anspruch 1, wobei der Grad der Krümmung und/oder Wendung so angepasst ist, dass er für mindestens eine der Vielzahl von kurvenförmigen Streben (108') unterschiedlich ist oder variiert.

4. Stent (100) nach Anspruch 1, wobei der Abstand zwischen benachbarten kurvenförmigen Streben (108') für mindestens ein Paar von benachbarten kurvenförmigen Streben (108') variiert.

5. Stent (100) nach Anspruch 1, wobei das dritte Stentzellenmuster des Übergangsabschnitts (106) ferner eine Vielzahl von geraden Streben (108) umfasst und ein Abstand zwischen benachbarten geraden Streben (108) die Zellen des Übergangsabschnitts (106) und des dritten Stentzellenmusters definiert.

6. Stent (100) nach Anspruch 5, wobei die Vielzahl von geraden Streben (108) in Bezug auf den inneren Klappenträgerabschnitt (104) geneigt ist und wobei benachbarte geneigte gerade Streben angepasst sind, um ineinander verschachtelt zu sein, wenn der Stent zusammengefaltet ist.

7. Stent (100) nach Anspruch 1, wobei der Übergangsabschnitt (106) mindestens eine Ausdehnungsform umfasst, die ausgewählt ist aus der Gruppe, die aus Folgenden besteht: kreisförmig, oval, elliptisch oder D-förmig.

8. Stent (100) nach Anspruch 1, wobei der Übergangsabschnitt (106) ein aufgeweitetes Profil umfasst, das einen Durchmesser umfasst, der gleich einem maximalen Ausdehnungsdurchmesser des äußeren Abschnitts (102) des Stents (100) oder größer als dieser ist.

9. Stent (100) nach Anspruch 1, wobei der Übergangsabschnitt (106) eine Vielzahl von keulenförmigen Elementen umfasst.

10. Stent (100) nach Anspruch 9, wobei der Stent angepasst ist, um eine native Herzklappe zu behandeln, die native Klappensegel umfasst, und wobei die Anzahl der keulenförmigen Elemente an eine Anzahl der nativen Klappensegel in der nativen Klappe angeglichen ist.

11. Stent (100) nach Anspruch 9, wobei die keulenförmigen Elemente des Übergangsabschnitts (106) angepasst sind, um Blut, das in einer regurgitierenden Richtung stromaufwärts durch die native Herzklappe über native Klappensegel fließt, auf eine Weise zu leiten, die das Schließvolumen und/oder den Schließdruck reduziert, das/der erforderlich ist, um die prothetischen Klappensegel, die innerhalb des inneren Klappenträgerabschnitts (104) angebracht sind, zu schließen.

12. Stent (100) nach Anspruch 1, wobei der äußere Abschnitt (102) einen oberen Abschnitt mit einer Ausdehnungsform aufweist, die aus der Gruppe ausgewählt ist, die aus Folgenden besteht: flach, konvex, konkav oder geneigt.

13. Stent (100) nach Anspruch 1, ferner umfassend:
mindestens zwei Schlitze (202), die durch benachbarte Streben definiert und gebildet sind, die das zweite Zellenstrukturmuster des inneren Klappenträgerabschnitts (104) bilden, wobei jeder Schlitz angepasst ist, um ein inneres Ende mindestens eines der prothetischen Klappensegel durch diesen hindurch aufzunehmen und ferner angepasst ist, um das prothetische Klappensegel innerhalb des inneren Klappenträgerabschnitts (104) auszurichten und die Ausrichtung aufrechtzuerhalten.

14. Stent (100) nach Anspruch 13, ferner umfassend:
eine oder mehrere Ösen (204) durch jede benachbarte Strebe, die jeden der mindestens zwei Schlitze (202) definieren und bilden, wobei jede Öse angepasst ist, um das innere Ende des prothetischen Klappenflügels mit diesem zu vernähen.

15. Stent (100) nach Anspruch 13, ferner umfassend:
drei Ösen (204) durch jede benachbarte Strebe, die jeden der mindestens zwei Schlitze (202) bilden und definieren,
wobei die drei Ösen (204) in jeder benachbarten Strebe drei Paare von Ösen definieren und
wobei ein Schlitz (202) zwischen den benachbarten Streben und den drei Paaren von Ösen, die durch die benachbarten Streben definiert sind, positioniert ist.

16. Stent (100) nach Anspruch 1,
wobei der äußere Abschnitt (102) eine Vielzahl von Streben umfasst, die das erste Stentzellenmuster definiert, und
wobei mindestens eine Gewebeanbringungsöse oder -Öffnung durch mindestens eine der Vielzahl von Streben des äußeren Abschnitts (102) definiert ist.

17. Stent (100) nach Anspruch 1, wobei der Stent (100) eine prothetische Mitralklappe oder eine prothetische Trikuspidalklappe umfasst.

18. Stent (100) nach Anspruch 1, wobei der äußere Abschnitt (102), der Übergangsabschnitt (106) und der innere Klappenträgerabschnitt (104) eine durchgehende Struktur umfassen.

## Revendications

1. Stent pouvant s'affaisser et s'expanser (100) conçu pour traiter une régurgitation du flux sanguin dans une valvule cardiaque native en permettant un flux sanguin en aval et en empêchant un flux sanguin en amont, le stent comprenant :
une section externe (102) comprenant un premier motif de cellules de stent ;
une section de support de valvule interne (104) s'étendant radialement vers le haut dans la section externe (102) et comprenant le premier motif de cellules de stent ou un deuxième motif de cellules de stent, la section de support de valvule interne (104) supportant des feuillets de valvule prothétique fixés dans celle-ci ;
une section de transition (106) entre la section externe (102) et la section de support de valvule interne (104) comprenant un troisième motif de cellules de stent qui est différent du premier motif de cellules de stent et du deuxième motif de cellules de stent, dans lequel la section de transition (106) tourne radialement vers l'intérieur pour donner lieu à une extension radialement vers le haut de la section de support de valvule interne (104) à l'intérieur de la section externe (102),
dans lequel le troisième motif de cellules de stent de la section de transition (106) comprend en outre une pluralité d'entretoises curvilignes (108') comprenant un degré de courbure et/ou de torsion,
dans lequel un espacement entre des entretoises curvilignes adjacentes (108') définit les cellules de la section de transition (106) et le troisième motif de cellules de stent, et
dans lequel des entretoises curvilignes adjacentes (108') sont configurées pour s'emboîter ensemble lorsque le stent (100) est affaissé.

2. Stent (100) selon la revendication 1,
dans lequel le degré de courbure et/ou de torsion est adapté pour être le même pour chacune de la pluralité d'entretoises curvilignes (108'), et
dans lequel l'espacement entre des entretoises curvilignes adjacentes (108') est constant ou égal à travers la pluralité d'entretoises curvilignes (108').

3. Stent (100) selon la revendication 1, dans lequel le degré de courbure et/ou de torsion est adapté pour différer ou varier pour au moins l'une de la pluralité d'entretoises curvilignes (108').

4. Stent (100) selon la revendication 1, dans lequel l'espacement entre des entretoises curvilignes adjacentes (108') varie pour au moins une paire d'entretoises curvilignes adjacentes (108').

5. Stent (100) selon la revendication 1, dans lequel le troisième motif de cellules de stent de la section de transition (106) comprend en outre une pluralité d'entretoises droites (108) et un espacement entre des entretoises droites adjacentes (108) définissant les cellules de la section de transition (106) et le troisième motif de cellules de stent.

6. Stent (100) selon la revendication 5, dans lequel la pluralité d'entretoises droites (108) sont inclinées par rapport à la section de support de valvule interne (104), et dans lequel des entretoises droites inclinées adjacentes sont adaptées pour s'emboîter ensemble lorsque le stent est affaissé.

7. Stent (100) selon la revendication 1, dans lequel la section de transition (106) comprend au moins une forme expansée choisie dans le groupe constitué par : une forme circulaire, ovale, elliptique ou en D.

8. Stent (100) selon la revendication 1, dans lequel la section de transition (106) comprend un profil évasé dont le diamètre est supérieur ou égal au diamètre maximal expansé de la section externe (102) du stent (100).

9. Stent (100) selon la revendication 1, dans lequel la section de transition (106) comprend une pluralité d'éléments en forme de lobe.

10. Stent (100) selon la revendication 9, dans lequel le stent est adapté pour traiter une valvule cardiaque native comprenant des feuillets natifs et dans lequel le nombre d'éléments en forme de lobe correspond à un nombre de feuillets natifs dans la valvule native.

11. Stent (100) selon la revendication 9, dans lequel les éléments en forme de lobe de la section de transition (106) sont adaptés pour diriger le sang s'écoulant dans une direction amont de régurgitation à travers la valvule cardiaque native à travers les feuillets natifs de manière à réduire le volume et/ou la pression de fermeture requis(e) pour fermer les feuillets de valvule prothétique fixés dans la section de support de valvule interne (104).

12. Stent (100) selon la revendication 1, dans lequel la section externe (102) présente une section supérieure de forme expansée choisie dans le groupe constitué par : une forme plate, convexe, concave ou inclinée.

13. Stent (100) selon la revendication 1, comprenant en outre :
au moins deux fentes (202) définies et formées par des entretoises adjacentes formant le deuxième motif de structure cellulaire de la section de support de valvule interne (104), chaque fente étant conçue pour recevoir une extrémité interne d'au moins l'un des feuillets de valvule prothétique à travers celle-ci et étant en outre conçue pour aligner et maintenir l'alignement du feuillet de valvule prothétique à l'intérieur de la section de support de valvule interne (104).

14. Stent (100) selon la revendication 13, comprenant en outre :
un ou plusieurs oeillets (204) traversant chaque entretoise adjacente définissant et formant chacune des au moins deux fentes (202), chaque oeillet étant adapté pour suturer l'extrémité interne du feuillet de valvule prothétique à celui-ci.

15. Stent (100) selon la revendication 13, comprenant en outre :
trois oeillets (204) traversant chaque entretoise adjacente qui forment et définissent chacune des au moins deux fentes (202),
dans lequel les trois oeillets (204) de chaque entretoise adjacente définissent trois paires d'oeillets, et
dans lequel une fente (202) est positionnée entre les entretoises adjacentes et les trois paires d'oeillets définies par les entretoises adjacentes.

16. Stent (100) selon la revendication 1,
dans lequel la section externe (102) comprend une pluralité d'entretoises définissant le premier motif de cellules de stent, et
dans lequel au moins un oeillet ou une ouverture de fixation de tissu est défini(e) à travers au moins l'une de la pluralité d'entretoises de la section externe (102).

17. Stent (100) selon la revendication 1, dans lequel le stent (100) comprend une valvule mitrale prothétique ou une valvule tricuspide prothétique.

18. Stent (100) selon la revendication 1, dans lequel la section externe (102), la section de transition (106) et la section de support de valvule interne (104) comprennent une structure continue.
